# EUROPÄISCHE PATENTSCHRIFT

(11) **EP 1 087 709 B1**
(45) Veröffentlichungstag und Bekanntmachung des Hinweises auf die Patenterteilung: **19.01.2005**
(21) Anmeldenummer: 99929232.9
(22) Anmeldetag: 16.06.1999
(51) Int. Cl.: A61B 17/32, A61F 9/007

(54) **CHIRURGISCHES INSTRUMENT ZUR GLEICHZEITIGEN ODER INTERMITTIERENDEN ABSTRAHLUNG VON LASERLICHT UND ULTRASCHALL**
SURGICAL INSTRUMENT FOR CONCURRENTLY OR INTERMITTENTLY EMITTING LASER LIGHT AND ULTRASOUND
INSTRUMENT CHIRURGICAL POUR EMETTRE EN CONTINU OU PAR INTERMITTENCE UNE LUMIERE LASER ET DES ULTRASONS

(30) Priorität: 17.06.1998 DE 19826912
(43) Veröffentlichungstag der Anmeldung: 04.04.2001
(73) Patentinhaber: Geuder AG, 69126 Heidelberg (DE)
(72) Erfinder: MÜLLER, Gerhard, D-14129 Berlin (DE); HARTMANN, Christian, D-14193 Berlin (DE); DESINGER, Kai, D-12157 Berlin (DE); SCHRÜNDER, Stephan, D-12163 Berlin (DE)
(74) Vertreter: Naumann, Ulrich, Dr.-Ing.
(86) Internationale Anmeldenummer: PCT/EP1999/004149
(87) Internationale Veröffentlichungsnummer: WO 1999/065405

(56) Entgegenhaltungen:
- WO-A-98/16157
- WO-A-99/13786
- DE-A- 4 322 955
- US-A- 4 729 373
- US-A- 5 397 293
- US-A- 5 702 360

## Beschreibung

Die Erfindung betrifft ein chirurgisches Instrument gemäß Oberbegriff der nebengeordneten Patentansprüche 1 und 13.

In der Augenchirurgie wird bei einer Katarakt zur Phakoemulsifikation mittels eines handgehaltenen Ultraschallschwingers, der distal in ein konzentrisches Saug-Spül-System integriert ist, der harte Linsenkern fragmentiert und abgesaugt. Je nach Konsistenz des Linsenkernes sind hierzu relativ hohe Ultraschallenergien notwendig,
so daß in der Regel erhebliche Zug- und Druckkräfte nicht nur auf die Linsenkapsel, sondern auch auf die die Kapsel aufspannenden Zonularfasern ausgeübt werden, mit der Folge, daß diese in der Regel in stärkerem Maße beschädigt werden.

Relativ häufig tritt jedoch auch der Fall auf, daß sich sehr harte bzw. extrem zähe Linsenkerne nicht vollständig fragmentieren und entfernen lassen, so daß die übliche Implantation von Fixfokus-Intraokularlinsen erschwert wird bzw. nur nach vollständiger Entfernung der Linsenkapsel möglich ist.

Es ist bekannt, daß mit gepulster Laserstrahlung ebenfalls eine Fragmentierung des Linsenkernes möglich ist. Hierzu werden beispielsweise von der Firma Aesculap Meditec gepulste Erbium-YAG-Laser eingesetzt. Es hat sich jedoch gezeigt, daß auch mit gepulsten Erbium-YAG-Lasern eine vollständige Fragmentierung und Absaugung der Linsenkeme schwierig ist, in der Regel jedenfalls aber deutlich länger dauert als mit dem beschriebenen Ultraschall-Phakoemulsifikationssystem.

Ein chirurgisches Instrument zur gleichzeitigen oder intermittierenden Abstrahlung von Ultraschall und Laserlicht, wie es eingangs beschrieben wurde, ist aus der Druckschrift DE 43 22 955 A1 bekannt. Der erzeugte Ultraschall wird bei diesem Instrument über einen optischen Wellenleiter übertragen, der gleichzeitig oder intermittierend zur Übertragung von in der Regel kontinuierlichem (cw-) Laserlicht verwendet wird. Zur Erzeugung des Ultraschalls wird ein Schallkopf mit zwei piezokeramischen Scheiben verwendet.

Alstechnische Lösung des Problems der Einkopplung der erzeugten Ultraschallwelle in den optischen Wellenleiter unter Anpassung der lateralen Ausdehnung der Welle an den Querschnitt des Wellenleiters und unter Vergrößerung ihrer Amplitude wird in der genannten Schrift ein als "getaperte" Faser ausgebildeter, optischer Wellenleiter offenbart: eine Quarzglasfaser ist mit ihrem proximalen Ende über einen Impedanzwandler an das distale Ende eines Ultraschallwandlers gekoppelt. Die Quarzglasfaser weist dabei an ihrem proximalen Ende einen an die Maße des Impedanzwandlers und der piezokeramischen Scheiben des Ultraschallwandlers angepaßten, erweiterten Querschnitt auf. Durch einen kürzeren, auch als Taper bezeichneten axialen Abschnitt mit sich in distaler Richtung exponentiell verjüngendem Längsschnittprofil wird ein Übergang zur Normaldicke der Glasfaser hergestellt und gleichzeitig die erforderliche Anpassung der lateralen Ausdehnung der Ultraschallwelle an die Faser sowie eine Vergrößerung der Amplitude der Ultraschallwelle erzielt.

Diese Lösung erweist sich in der Praxis jedoch als außerordentlich problematisch, da zum einen die Herstellungskosten derartiger Taper besonders hoch sind und zum anderen die Fusionsstelle zwischen dem distalem Ende des Tapers und der anschließenden, regulären optischen Faser sehr bruchsensitiv ist.

Ein weiteres chirurgisches Instrument der gattungsbildenden Art ist aus der US 47 29 373 bekannt. Das Laserlicht wird bei diesem Instrument in einen optischen Wellenleiter eingekoppelt, welcher sich innerhalb eines Gehäuses erstreckt. Der optische Wellenleiter mündet in eine mittels eines Verbindungsstücks fest mit dem optischen Wellenleiter verbundene kristalline Spitze. Mittels innerhalb des Gehäuses angeordneter Ultraschallwandler kann ein den optischen Wellenleiter umgebender Ring in Schwingung versetzt werden. Durch eine feste Kopplung des Rings mit dem optischen Wellenleiter, welches wiederum fest mit der kristallinen Spitze gekoppelt ist, können die Ultraschallschwingungen auf die kristalline Spitze übertragen werden. Dieses Instrument erweist sich dahingehend als problematisch, dass die Ultraschallschwingungen Rückwirkungen auf die Laserankopplung ausüben können. Ein störungsfreier Betrieb des Instruments ist somit nicht hinreichend gewährleistet.

Aufgabe der Erfindung ist es nunmehr, ein chirurgisches Instrument der eingangs genannten Art anzugeben, das besonders einfach herstellbar und unanfälliger für Störungen ist.

Erfindungsgemäß wird die voranstehende Aufgabe zum einen durch die Merkmale des Patentanspruchs 1 gelöst. Danach ist das in Rede stehende chirurgische Instrument derart ausgebildet, dass das Laserlicht über einen gehäusefesten Anschluss und einen im Gehäuse geführten optischen Wellenleiter in den akustooptischen Wellenleiter eingekoppelt wird, dass der optische Wellenleiter durch eine sich koaxial mit dem akusto-optischen Wellenleiter im Ultraschallwandler erstreckende Bohrung verläuft und dass der optische und der akusto-optische Wellenleiter in axialer Richtung beabstandet sind.

In erfindungsgemäßer Weise ist erkannt worden, dass ein störungsfreier Betrieb des chirurgischen Instruments weitestgehend sichergestellt ist, wenn der optische Wellenleiter akustisch entkoppelt wird. Zur Kopplung wird erfindungsgemäß eine Beabstandung der einander zugewandten Enden des optischen und des akustooptischen Wellenleiters in axialer Richtung vorgeschlagen. Durch diese Luftbrücke zwischen dem letzten optischen Glied der Laserstrahlen übertragenden Einrichtung und der die Ultraschallenergie transportierenden Faser werden Rückwirkungen der Ultraschallschwingungen auf die Laserstrahleinkopplung in die Stimfläche des akusto-optischen Wellenleiters effektiv vermieden.

Erfindungsgemäß wird die Aufgabe darüber hinaus durch die Merkmale des nebengeordneten Patentanspruchs 13 gelöst. Danach ist das in Rede stehende chirurgische Instrument derart ausgebildet, dass proximal innerhalb des Gehäuses ein Laser angeordnet ist, der einen mit dem akusto-optischen Wellenleiter koaxialen Lichtstrahl erzeugt, und dass durch den Ultraschallwandler ein optischer Wellenleiter geführt ist, an dessen proximalem Ende das Laserlicht mit Hilfe einer Sammellinse eingekoppelt wird, wobei der optiscne und der akusto-optische Wellenleiter in axialer Richtung beabstandet sind.

In erfindungsgemäßer Weise ist erkannt worden, dass ein störungsfreier Betrieb des chirurgischen Instruments auch dann sichergestellt ist, wenn der Laser innerhalb des Gehäuses proximal angeordnet ist. Zur Einkopplung in den optischen Wellenleiter passiert der Lichtstrahl dabei erfindungsgemäß eine Sammellinse, in deren Brennebene das proximale Ende des optischen Wellenleiters angeordnet ist. Zur akustischen Einkopplung des optischen Wellenleiters sind der optische und der akusto-optische Welleleiter in axialer Richtung beabstandet. Die interne Laserquelle - vorzugsweise kommt ein Kleinstlaser, etwa ein Halbleiterlaser, in Frage - hat den weiteren Vorteil, dass sie frei handhabbar ist, da kein externer optischer Wellenleiter an das Gehäuse angeschlossen werden muss.

Bei einer Ausführungsform der Erfindung ist der Ultraschallwandler ein piezoelektrisch betriebener Verbundwandler mitaxialen Schwingmassen. DerSchwingkörper bildet zumindest einen Teil der distalen Schwingmasse des Verbundwandlers. Bei dieser Ausbildung des Ultraschallwandlers wird im wesentlichen auf bekannte, weit verbreitete und daher leicht und günstig beschaffbare Bauelemente zurückgegriffen.

Mindestens die distale Schwingmasse des Verbundwandlers weist bei einer bevorzugten Ausführungsform der Erfindung in axialer Richtung mehrere Abschnitte auf. Zumindest einer dieser axialen Abschnitte ist als Amplitudentransformator, vorzugsweise als Stufenhom ausgebildet. Die Amplitude der elektrisch angeregten Schwingung der piezokeramischen Scheiben des Ultraschallwandlers kann so mit einfachen, herkömmlichen Methoden auf den für die chirurgische Anwendung notwendigen Wert vergrößert werden.

Bei einem anderen, bevorzugten Ausführungbeispiel der Erfindung hat der Schwingkörper ein zu seinem distalen Ende hin in axialer Richtung des akustooptischen Wellenleiters sich etwa kegelförmig zuspitzendes, lösbares und durch eine formschlüssige Verbindung befestigtes Koppelelement, in dem eine von der Kegelspitze her längs der Kegelachse verlaufende und mit dem akusto-optischen Wellenleiter koaxiale Bohrung zur Aufnahme des proximalen Endabschnitts des Wellenleiters ausgebildet ist. Dieserwird vorzugsweise mit Hilfe eines Epoxidharzes eingeklebt. In Versuchsreihen mit auf dem Markt erhältlichen Zwei-Komponenten-Reaktionsklebern auf Epoxidharz-Basis wurde ermittelt, daß geeignete derartige Klebstoffe heiß aushärtend sind und sich durch eine Glastemperatur von mehr als 100 °C sowie eine geringe akustische Dämpfung auszeichnen. Die geringe akustische Dämpfung fördert die möglichst verlustfreie Einkopplung der erzeugten Ultraschallwelle in die Glasfaser zur Weiterleitung an die Applikationsstelle. Die hohe Glastemperatur ist erforderlich, damit die beim Betrieb des Instruments auftretende Erwärmung des Wellenleiters den Klebstoff nicht erweicht. Durch einen relativ weichen Kleber würden die Schallübertragungseigenschaften des Klebstoffes stark beeinträchtigt, seine akustische Dämpfung also wesentlich erhöht.

Mit einer derartigen Anordnung sich läßt sich Leistungsultraschall überraschenderweise auch bis zu Frequenzen von 100 kHz, typischerweise 50 kHz, verlustfrei übertragen.

Der Abstand zwischen dem optischen und dem akusto-optischen Wellenleiter beträgt typischerweise zwischen 50 und 800 Mikrometer.

Die distale Faser wird bei einer insbesondere für die Phakoemulsifikation geeigneten Ausführungsform in ein konzentrisches Saug-Spül-System integriert, das vorzugsweise eine ovale bis elliptische Querschnittsform aufweist, wobei im Zentrum die Ultraschall und Laserlicht führende Quarzfaser angeordnet ist und in denen sich diametral gegenüberliegende randständige "Zwickel" der Saug- und Spül-Kanäle angeordnet sind. Eine derartige, ovale bis elliptische Ausformung des distalen Saug-Spül-Systems mit kombinierter Laserultraschallfaser hat den Vorteil, daß mit einer nach dem Stand der Technik üblichen Längsinzision in der Nähe des Äquators der Linsenkapsel ein minimales Zugtrauma auf die Linsenkapsel und damit die Zonularfasern ausgeübt wird, gleichzeitig aber hinreichende Strömungsquerschnitte für das Saug-Spül-System möglich sind. Mit einem derartigen Kombinationsapplikator ist es erstmals möglich, simultan, jedoch getrennt voneinander Ultraschall- und Laserenergie so einzustellen, daß ein minimales Zug- und Drucktrauma auf die Linsenkapsel und damit die Zonularfasern ausgeübt wird und die partiellen Vorteile beider Energieformen zur Phakoemulsifikation kombiniert unter Beibehaltung der minimalen Raumforderung der Einzelapplikationssysteme eingesetzt werden können.

Weitere Merkmale und Vorteile der Erfindung werden bei der nachfolgenden Beschreibung zweier Ausführungsbeispiele anhand der Zeichnung verdeutlicht. Darin zeigen
- Figur 1: eine vereinfachte Längsschnittansicht eines Ultraschall-Laser-Kombihandstücks, bei dem die Laserstrahlung über eine proximal zugeführte Lichtleitfaser eingekoppelt wird,
- Figur 2: eine vergrößerte Ansicht des distalen Endes des Ultraschallwandlers aus Figur 1 zur Verdeutlichung der Verbindung mit dem akustooptischen Wellenleiter,
- Figur 3: eine alternative Ausführung eines Ultraschall-Laser-Handstücks, bei der die Laserstrahlung im Instrument selbst generiert wird,
- Figur 4: eine Längsschnittansicht des distalen Endes des Instruments zur Erläuterung des Saug-Spül-Systems und
- Figur 5: eine perspektivische Ansicht der distalen Instrumentspitze.

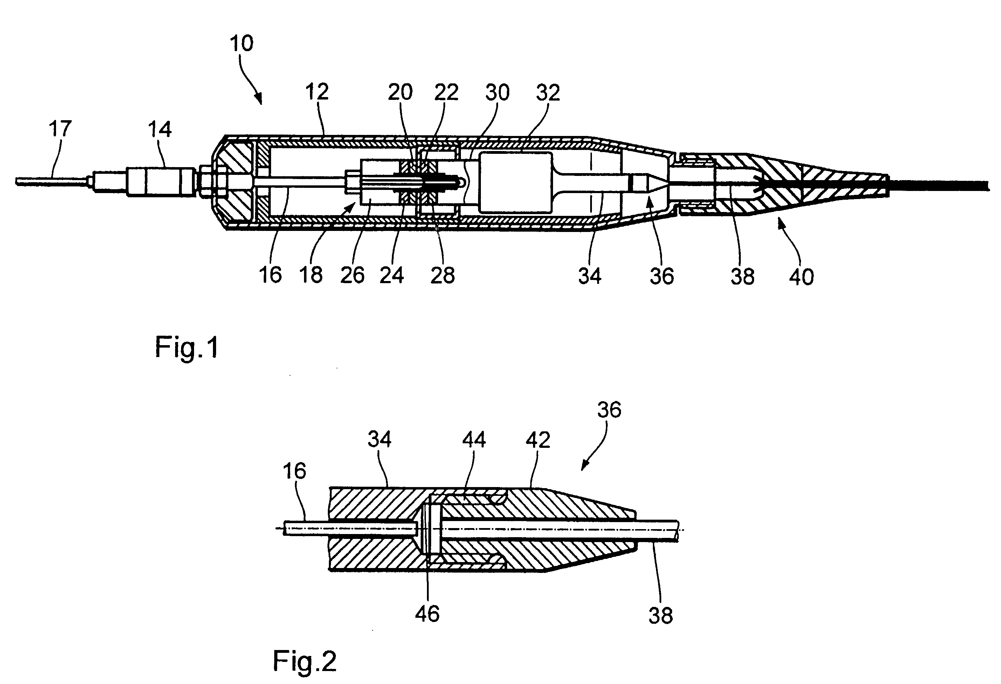

Abbildung 1 zeigt in einer vereinfachten Längsschnittansicht den Aufbau eines Applikators 10 für die kombinierte Laser-/Ultraschallbehandlung. Sein Gehäuse 12 ist im wesentlichen symmetrisch um eine Achse ausgebildet, längs derer sich ein proximal mit einem Anschluß für einen modifizierten SMA-Stecker 14 verbundener interner optischer Wellenleiter 16 innerhalb des Gehäuses 12 erstreckt. Von extern ist ein weiterer optischer Wellenleiter 17 mit Hilfe des modifizierten SMA-Steckers 14 angeschlossen. Nicht dargestellt in Figur 1 ist eine externe Laserquelle, deren Licht in den externen Wellenleiter 17 mit Hilfe geeigneter optischer Komponenten eingekoppelt wird. Der interne optische Wellenleiter 16 ist typischerweise aus Quarzglas ausgebildet und hat bei diesem Ausführungsbeispiel einen Durchmesser von etwa 380 Mikrometern. Die Quarzglasfaser ist für die Lichtleitung im Spektralbereich zwischen dem nahen Ultraviolett (UV) und dem nahen Infrarot (IR) geeignet. Ebenso kann jedoch bei Verwendung einer geeigneten optischen Faser aus beispielsweise Zirkonfluorid Strahlung im mittleren IR-Spektralbereich zwischen 2,6 und 3 µm zum Einsatz gelangen. Zur Erzeugung dieser Strahlung kann ein Erbium:Yttriumaluminiumgranat (Er:YAG)-Laser verwendet werden. Im nahen UV kommt beispielsweise ein gepulster Xenonchlorid- bzw. Argonfluorid-Excimerlaser zur Anwendung, der bei einer Wellenlänge von 308 bzw. 190 nm arbeitet. Seine gepulste Strahlung hat Impulsdauern zwischen 100 und 200ns. Es kann jedoch auch die Strahlung eines bei Bedarf frequenzvervielfachten Festkörperlasers, etwa eines Nd:YAG-Lasers, mit Impulsdauern zwischen 20 ns und 300 ps verwendet werden.

Der optische Wellenleiter 16 wird distal durch eine zentrale Bohrung eines Ultraschallwandlers 18 geführt. Dieser ist in Figur 1 teilweise geöffnet dargestellt, um die Führung des Wellenleiters 16 zu verdeutlichen. Zum Schutz des optischen Wellenleiters 16 gegen erzeugten Ultraschall ist er von einer Edelstahlhülse und einem Teflonmantel umgeben.

Der Ultraschallwandler 18 ist als piezoelektrisch betriebener Verbundschwinger ausgeführt. Seine piezokeramischen Scheiben 20 und 22 sind in axialer Richtung jeweils mit Schwingmassen 24,26 bzw. 28 und 30 verbunden. Die Schwingmassen sind zur Erzeugung einer hohen Ultraschalleistung aus Titan oder einer Titanlegierung gefertigt, können aber bei entsprechend geringerem Leistungsbedarf auch aus einer Aluminiumlegierung oder kristallinem Silizium bestehen. Distal schließt sich an die Schwingmasse 30 ein als Stufenhom ausgebildeter Amplitudentransformätor 32 an. An einem zylindrischen Abschnitt 34 ist distal ein kegelförmig zulaufendes Koppelstück 36 befestigt. Einzelheiten des Koppeistücks 36 werden unten anhand von Figur 2 beschrieben.

In das Koppelstück 36 ist ein akusto-optischer Wellenleiter 38 eingeklebt. Der akusto-optische Wellenleiter besteht aus einem Quarzglas- oder Saphirkern, einem Cladding sowie einem Kunststoffmantel aus Acrylat, Polyacrylat oder Polyimid. Diese Mantelmaterialien eignen sich besonders, da sie eine geringe akustische Dämpfung aufweisen. Der Außendurchmesser des akusto-optischen Wellenleiters 38 liegt je nach Anwendung zwischen etwa 400 und 1000 µm, typischerweise bei etwa 600 µm. Seine Länge beträgt typischerweise etwa 5 cm, so daß sich allfällige Restabsorptionen des verwendeten Materials für die jeweils verwendete Lichtwellenlänge praktisch nicht auswirken.

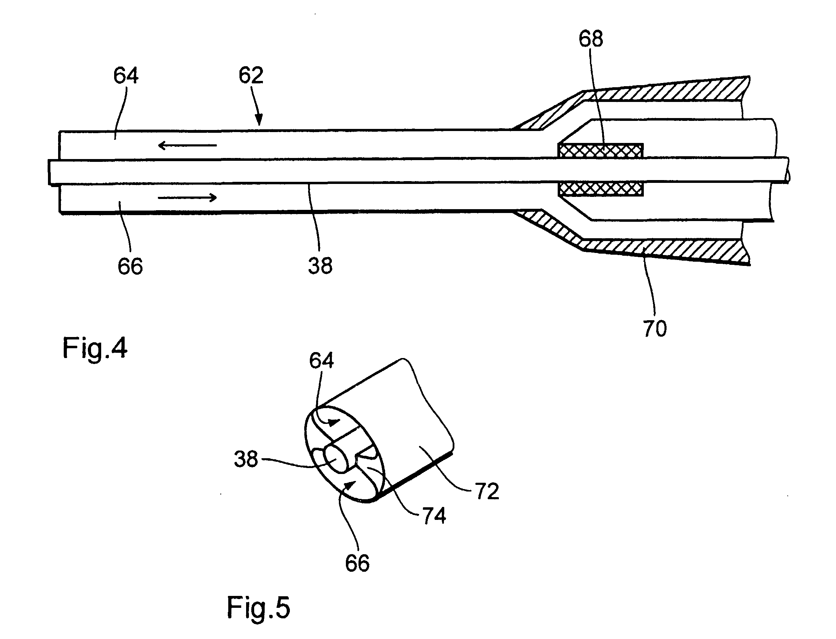

Das distale Ende des Applikators 10 bildet ein steril auswechselbares, je nach Anwendung ausgebildetes Endstück 40, durch dessen zentrale Öffnung derakustooptische Wellenleiter 38 aus dem Gehäuse 12 nach außen tritt. Nähere Einzelheiten des Endstücks 40 werden anhand der Figuren 4 und 5 beschrieben.

Figur 2 zeigt in einer vergrößerten Längsschnittdarstellung das Koppelstück 36. Es hat proximal einen kurzen zylindrischen Abschnitt 42, der über eine lösbare, formschlüssige Verbindung 44 am zylindrischen Abschnitt 34 befestigt ist. Die Verbindung 44 ist hier als Schraubverbindung ausgeführt. Distal verjüngt sich das Koppelstück etwa kegelförmig bis hin zu seiner Spitze, von der aus sich eine Bohrung längs der Achse des Koppelstücks 36 bis hin zum zylindrischen Abschnitt 34 erstreckt. Mit der Wandung dieser Bohrung ist der akusto-optische Wellenleiter 38 mit Hilfe eines Epoxidharz-Klebers fest verbunden.

Der zylindrische Abschnitt 34 hat eine zentrische Bohrung, durch die der optische Wellenleiter 16 vom Ultraschallwandler 18 her zum askusto-optischen Wellenleiter 38 hingeführt wird. Zwischen der distalen Stirnfläche des optischen Wellenleiters 16 und der proximalen Stirnfläche des akusto-optischen Wellenleiters 38 ist ein in seiner axialen Länge definiert einstellbarer Luftspalt 46 von ca. 50 bis 800 µm vorgesehen, um Rückwirkungen der Ultraschallschwinung des akusto-optischen auf den optischen Wellenleiter zu vermeiden.

Durch die beschriebene Gestaltung des Koppelelements 36 kann es mit dem akustooptischen Wellenleiter 38 zusammen bei Bedarf nach ein- oder mehrmaliger Benutzung in einfacher Weise abgeschraubt und ausgetauscht werden, um durch ein entsprechendes, anderen Anforderungen angepaßtes Koppelstück ersetzt zu werden. Auch bei etwaiger Beschädigung des Wellenleiters 38 kann schnell ein Ersätzstück angeschraubt werden.

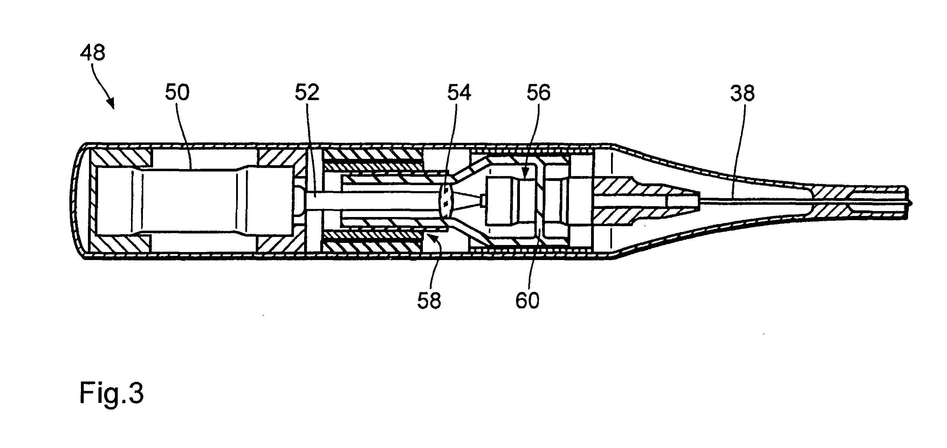

Figur 3 zeigt einen Applikator 48 in einer teilweise geschnittenen Längsschnittansicht. Sein Aufbau ist grundsätzlich dem des oben beschriebenen Applikators 10 ähnlich und wird daher im folgenden mit Blick lediglich auf wesentliche Unterschiede erläutert.

In den Applikator 48 ist proximal eine Laserbaugruppe 50 integriert, so daß in diesem Abschnitt ein optischer Wellenleiter entfallen kann. Das von der Laserbaugruppe 50 emittierte Licht, in Figur 2 durch ein paralleles Lichtbündel 52 repräsentiert, wird vielmehr frei durch das Gehäuse geleitet und passiert eine Sammellinse 54, in deren Brennpunkt die proximale Stirnfläche des akustooptischen Wellenleiters 38 angeordnet ist. Diese erstreckt sich also durch eine zentrische Bohrung vom proximalen Ende des Ultraschallwandlers 56 bis hin zum kegelförmig zugespitzten, distalen Ende des Koppelstücks. Die Einkopplung der Ultraschallwelle in den akusto-optischen Wellenleiter erfolgt jedoch wie oben beschrieben am distal kegelförmig zulaufenden Koppelstück.

Bei einer erfindungsgemäßen Ausführung ähnlich dem oben anhand der Figuren 1 und 2 geschilderten Ausführungsbeispiel wird durch den Ultraschallwandler 56 ein optischer Wellenleiter geführt, an dessem proximalen Ende das Laserlicht mit Hilfe der Linse 54 eingekoppelt wird. Im kegelförmig zulaufenden distalen Ende des Amplitudentransformators ist analog zur Darstellung in Figur 2 der akusto-optische Wellenleiter 38 mit seinem proximalen Ende befestigt. Zwischen dem proximalen Ende des akusto-optischen Wellenleiters und dem distalen Ende des optischen Wellenleiters ist ein Luftspalt ausgebildet.

Eine elektrische Motoreinheit 58 dient zur linearen Verschiebung des in einem Rahmen 58 beweglich gelagerten Ultraschallwandlers 56 in axialer Richtung. Am distalen Ende des Applikators 48 ist eine auswechselbare Aufnahme für ein Faserführungssystem sowie eine Spülung und Absaugung 62 vorgesehen, die im folgenden näher beschrieben werden.

Die Figuren 4 und 5 zeigen die Spitze des Instrumentes 48 bzw. 10 mit der Vorrichtung 62 zum Spülen und Absaugen eines Applikationsgebiets. Die Vorrichtung weist zwei separate Kanäle 64 und 66 auf, von denen einer (64) zum Spülen mit einer Kochsalzlösung und der andere (66) sowie zum Absaugen vorgesehen ist. Der Schaft der Vorrichtung 62 geht proximal über in eine schleusenartige Aufnahme 70, welche über eine Dichtung 68 den Saug- und Spülkanal vom restlichen Instrument abdichtet. Eine Faserführung 74 dient zur Positionierung des optoakustischen Wellenleiters 38.

Diese in den Figuren 4 und 5 dargestellte Einheit 62 ist vom Instrument 48 bzw. 10 einfach durch Verschrauben oder Einrasten zu trennen, um bei erneutem Einsatz des Instruments ein anderes, steriles Schleusensystem einsetzen zu können.

In Weiterführung des Erfindungsgedankens hat es sich als vorteilhaft herausgestellt, daß konzentrisch um das distale Ende der Laser und Ultraschall leitenden Therapiefaser angeordnete Saug-/Spülsystem optional auch als separates Handstück anzuordnen, um dadurch ein bimanuelles Hantieren zu ermöglichen. Dabei wird dann das distale Ende entweder wie bei einem der anhand von Figur 1 bis 3 beschriebenen Ausführungsbeispiele ausgeführt und die Laser und Ultraschall führende Faser zum Schutz vor zufälligen Knickbelastungen in einer endständigen Kanüle geführt. In dem zweiten Handstück sind dann konzentrisch bzw. paraxial die Zu- und Abführungen des Saug-/Spülsystems angeordnet. Bei Nutzung dieser erfindungsgemäßen Variante können die distalen Durchmesser der einzelnen Handstücke jeweils unter 0,6 mm bleiben und gleichzeitig kann der Operateur durch das bimanuelle Vorgehen die durch die Applikatoren eingebrachten Zug- und Scherkräfte weiter minimieren.

## Patentansprüche

1. Chirurgisches Instrument (10) zur gleichzeitigen oder intermittierenden Abstrahlung von Laserlicht und Ultraschall, mit einem Gehäuse (12), einem akustooptischen Wellenleiter (38) in dem Gehäuse, der durch eine distal ausgebildete Öffnung des Gehäuses (12) nach außen tritt und an dessen proximalem Ende Laserlicht einkoppelbar ist, einem Ultraschallwandler (18) innerhalb des Gehäuses (12) zur Erzeugung einer sich längs des Wellenleiters (38) ausbreitenden Ultraschallwelle, und einem proximal mit dem Ultraschallwandler (18) und distal mit dem Wellenleiter (38) verbundenen Schwingkörper (28 bis 36) zur Einkopplung der Ultraschallwelle in den Wellenleiter unter Anpassung der lateralen Ausdehnung der Ultraschallwelle an den Querschnitt des Wellenleiters (38) und unter Vergrößerung ihrer Amplitude, wobei der Schwingkörper (28 bis 36) eine zumindest an seinem distalen Ende ausgebildete, mit dem akusto-optischen Wellenleiter (38) koaxiale Bohrung hat, an deren Wandung der akusto-optische Wellenleiter (38) befestigt ist, **dadurch gekennzeichnet, dass** das Laserlicht über einen gehäusefesten Anschluss und einen im Gehäuse (12) geführten optischen Wellenleiter (16) in den aklusto-optischen Wellenleiter (38) eingekoppelt wird, dass der optische Wellenleiter (16) durch eine sich koaxial mit dem akusto-optischen Wellenleiter (38) im Ultraschallwandler (18) erstreckende Bohrung verläuft und dass der optische (16) und der akusto-optische Wellenleiter (38) in axialer Richtung beabstandet sind.

2. Chirugisches Instrument nach Anspruch 1, **dadurch gekennzeichnet, daß** der Ultraschallwandler (18, 56) ein piezoelektrisch betriebener Verbundwandler mit axialen Schwingmassen (24 bis 36) ist und daß der Schwingkörper (28 bis 36) zumindest einen Teil der distalen Schwingmasse des Verbundwandlers (18, 56) bildet.

3. Chirurgisches Instrument nach Anspruch 2, **dadurch gekennzeichnet, daß** die Schwingmassen (24 bis 36) aus Titan, einer Titanlegierung, einer Aluminiumlegierung oder aus kristallinem Silizium gefertigt sind.

4. Chirurgisches Instrument nach Anspruch 2 oder 3, **dadurch gekennzeichnet, daß** mindestens die distale Schwingmasse des Verbundwandlers (18, 56) in axialer Richtung mehrere Abschnitte (28 bis 36) aufweist, und daß zumindest ein axialer Abschnitt der distalen Schwingmasse des Ultraschallwandlers (18, 56) als Amplitudentransformator (32) ausgebildet ist.

5. Chirurgisches Instrument nach einem der vorhergehenden Ansprüche, **dadurch gekennzeichnet, daß** der Amplitudentransformator (32) ein Stufenhorn ist.

6. Chirurgisches Instrument nach einem der vorhergehenden Ansprüche, **dadurch gekennzeichnet, daß** der Schwingkörper ein zu seinem distalen Ende hin in axialer Richtung des akusto-optischen Wellenleiters sich etwa kegelförmig zuspitzendes, lösbares und durch eine formschlüssige Verbindung (44) befestigtes Koppelelement (36) hat, in dem die von der Kegelspitze her längs der Kegelachse verlaufende und mit dem akustooptischen Wellenleiter (38) koaxiale Bohrung zur Aufnahme dessen proximalen Endabschnitts ausgebildet ist.

7. Chirurgisches Instrument nach einem der vorhergehenden Ansprüche, **dadurch gekennzeichnet, daß** der akusto-optische Wellenleiter (38) im Bereich seines proximalen Endes mit Hilfe einer Klebeverbindung an der Wandung der distalen Bohrung des Koppefelementes (36) befestigt ist.

8. Chirurgisches Instrument nach einem der vorhergehenden Ansprüche, **dadurch gekennzeichnet, daß** die Klebeverbindung zwischen dem Wellenleiter (38) und den Wandungen der Bohrung durch einen heiß aushärtenden Epoxidharz mit einer hohen Glastemperatur und einer geringen akustischen Dämpfung gebildet wird.

9. Chirurgisches instrument nach Anspruch 8, **dadurch gekennzeichnet, daß** die Glastemperatur des Epoxidharzes mindestens 100 °C beträgt.

10. Chirurgisches Instrument nach einem der vorhergehenden Ansprüche, **dadurch gekennzeichnet, daß** der akusto-optische Wellenleiter (38) eine Quarzglasfaser mit einem Durchmesser zwischen 300 und 1700 Mikrometern ist.

11. Chirurgisches Instrument nach Anspruch 10, **dadurch gekennzeichnet, daß** der Wellenleiter (38) von einem Mantel aus Acrylat, Polyacrylat oder Polyimid umgeben ist.

12. Chirurgisches Instrument nach einem der Ansprüche 1 bis 11, **dadurch gekennzeichnet, daß** der Abstand (46) zwischen dem optischen (16) und dem akusto-optischen Wellenleiter (38) zwischen 50 und 800 Mikrometer beträgt.

13. Chirurgisches Instrument (48) zur gleichzeitigen oder intermittierenden Abstrahlung von Laserlicht und Ultraschall, mit einem Gehäuse (12), einem akustooptischen Wellenleiter (38) in dem Gehäuse, der durch eine distal ausgebildete Öffnung des Gehäuses (12) nach außen tritt und an dessen proximalem Ende Laserlicht einkoppelbar ist, einem Ultraschallwandler (56) innerhalb des Gehäuses (12) zur Erzeugung einer sich längs des Wellenleiters (38) ausbreitenden Ultraschallwelle, und einem proximal mit dem Ultraschallwandler (56) und distal mit dem Wellenleiter (38) verbundenen Schwingkörper (28 bis 36) zur Einkopplung der Ultraschallwelle in den Wellenleiter unter Anpassung der lateralen Ausdehnung der Ultraschallwelle an den Querschnitt des Wellenleiters (38) und unter Vergrößerung ihrer Amplitude, wobei der Schwingkörper (28 bis 36) eine zumindest an seinem distalen Ende ausgebildete, mit dem akusto-optischen Wellenleiter (38) koaxiale Bohrung hat, an deren Wandung der akusto-optische Wellenleiter (38) befestigt ist, **dadurch gekennzeichnet, dass** proximal innerhalb des Gehäuses ein Laser (50) angeordnet ist, der einen mit dem akusto-optischen Wellenleiter (38) koaxialen Lichtstrahl (52) erzeugt, und dass durch den Ultraschallwandler (56) ein optischer Wellenleiter geführt ist, an dessen proximalem Ende das Laserlicht mit Hilfe einer Sammellinse (54) eingekoppelt wird, wobei der optische und der akusto-optische Wellenleiter (38) in axialer Richtung beabstandet sind.

14. Chirurgisches Instrument nach einem der vorhergehenden Ansprüche, **dadurch gekennzeichnet, daß** der Ultraschallwandler (18, 56) innerhalb des Gehäuses (12) in axialer Richtung des akusto-optischen Wellenleiters (38) verschiebbar ist.

15. Chirurgisches Instrument nach Anspruch 14, **gekennzeichnet durch** einen Linearmotor (58) zum Antrieb der axialen Bewegung des Ultraschallwandlers.

16. Chirurgisches Instrument nach einem der vorhergehenden Ansprüche, insbesondere zur Anwendung bei der Phakoemulsifikation, **gekennzeichnet durch** einen vom restlichen Gehäuse (12) lösbaren, rohrförmigen Abschnitt (62) am distalen Ende des Gehäuses (12), mit mindestens zwei darin ausgebildeten Stegen (74) zur linearen Führung des akusto-optischen Wellenleiters, wobei **durch** die Stege (74) und den Wellenleiter das Rohr in mindestens zwei sich axial erstreckende und hinsichtlich des Flüssigkeitsaustauschs isolierte Kammern (64, 66) unterteilt ist, deren erste mit einer Saugvorrichtung und deren zweite mit einer Spülvorrichtung verbindbar ist.

17. Chirurgisches Instrument nach einem der vorhergehenden Ansprüche, **dadurch gekennzeichnet, daß** sein distales Ende als Einstichkanüle ausgebildet ist, in deren Innerem der akusto-optische Wellenleiter (38) in Schwingungsrichtung beweglich geführt ist.

## Claims

1. Surgical instrument (10) for the concurrent or intermittent emission of laser light and ultrasound, having a housing (12), an acousto-optical waveguide (38) in the housing, which waveguide exits through a distally formed aperature of the housing (12) and at the proximal end of which waveguide laser light can be coupled in, an ultrasonic transducer (18) inside the housing (12) for generating an ultrasonic wave which propagates along the waveguide (38), and a vibrating body (28 to 36), connected proximally to the ultrasonic transducer (18) and distally to the waveguide (38), for coupling the ultrasonic wave into the waveguide while adapting the lateral extent of the ultrasonic wave to the cross-section of the waveguide (38) and increasing its amplitude, the vibrating body (28 to 36) having a bore which is formed at least at its distal end and which is coaxial with the acousto-optical waveguide (38), to the wall of which bore the acousto-optical waveguide (38) is fastened, **characterised in that** the laser light is coupled into the acousto-optical waveguide (38) *via* a connection which is fixed to the housing and *via* an optical waveguide (16) which is guided in the housing (12), the optical waveguide (16) runs through a bore extending coaxially with the acousto-optical waveguide (38) in the ultrasonic transducer (18), and the optical waveguide (16) and the acousto-optical waveguide (38) are spaced apart in the axial direction.

2. Surgical instrument according to claim 1, **characterised in that** the ultrasonic transducer (18, 56) is a piezoelectrically operated composite transducer having axial vibrating masses (24 to 36), and the vibrating body (28 to 36) forms at least part of the distal vibrating mass of the composite transducer (18, 56).

3. Surgical instrument according to claim 2, **characterised in that** the vibrating masses (24 to 36) are made of titanium, a titanium alloy, an aluminium alloy or of crystalline silicon.

4. Surgical instrument according to claim 2 or 3, **characterised in that** at least the distal vibrating mass of the composite transducer (18, 56) has a plurality of sections (28 to 36) in the axial direction, and at least one axial section of the distal vibrating mass of the ultrasonic transducer (18, 56) is constructed as an amplitude transformer (32).

5. Surgical instrument according to any one of the preceding claims, **characterised in that** the amplitude transformer (32) is a stepped horn.

6. Surgical instrument according to any one of the preceding claims, **characterised in that** the vibrating body has a coupling element (36) which tapers approximately conically towards its distal end in the axial direction of the acousto-optical waveguide and which is detachable and is fastened by a positive connection (44), in which coupling element (36) the bore that extends from the tip of the cone along the axis of the cone and that is coaxial with the acousto-optical waveguide (38) is formed for receiving the proximal end portion of that waveguide (38).

7. Surgical instrument according to any one of the preceding claims, **characterised in that** the acousto-optical waveguide (38) is fastened in the region of its proximal end to the wall of the distal bore of the coupling element (36) by means of an adhesive bond.

8. Surgical instrument according to any one of the preceding claims, **characterised in that** the adhesive bond between the waveguide (38) and the walls of the bore is formed by a heat-curing epoxy resin exhibiting a high glass transition temperature and low acoustic attenuation.

9. Surgical instrument according to claim 8, **characterised in that** the glass transition temperature of the epoxy resin is at least 100°C.

10. Surgical instrument according to any one of the preceding claims, **characterised in that** the acousto-optical waveguide (38) is a silica glass fibre having a diameter of from 300 to 1700 micrometres.

11. Surgical instrument according to claim 10, **characterised in that** the waveguide (38) is surrounded by cladding composed of acrylate, polyacrylate or polyimide.

12. Surgical instrument according to any one of claims 1 to 11, **characterised in that** the spacing (46) between the optical waveguide (16) and the acousto-optical waveguide (38) is from 50 to 800 micrometres.

13. Surgical instrument (48) for the concurrent or intermittent emission of laser light and ultrasound, having a housing (12), an acousto-optical waveguide (38) in the housing, which waveguide exits through a distally formed aperature of the housing (12) and at the proximal end of which waveguide laser light can be coupled in, an ultrasonic transducer (56) inside the housing (12) for generating an ultrasonic wave which propagates along the waveguide (38), and a vibrating body (28 to 36), connected proximally to the ultrasonic transducer (56) and distally to the waveguide (38), for coupling the ultrasonic wave into the waveguide while adapting the lateral extent of the ultrasonic wave to the cross-section of the waveguide (38) and increasing its amplitude, the vibrating body (28 to 36) having a bore which is formed at least at its distal end and which is coaxial with the acousto-optical waveguide (38), to the wall of which bore the acousto-optical waveguide (38) is fastened, **characterised in that** a laser (50) is arranged proximally inside the housing, which laser (50) generates a light beam (52) which is coaxial with the acousto-optical waveguide (38), and an optical waveguide is guided through the ultrasonic transducer (56), at the proximal end of which optical waveguide the laser light is coupled in by means of a focusing lens (54), the optical waveguide and the acousto-optical waveguide (38) being spaced apart in the axial direction.

14. Surgical instrument according to any one of the preceding claims, **characterised in that** the ultrasonic transducer (18, 56) is displaceable inside the housing (12) in the axial direction of the acousto-optical waveguide (38).

15. Surgical instrument according to claim 14, **characterised by** a linear motor (58) for driving the axial movement of the ultrasonic transducer.

16. Surgical instrument according to any one of the preceding claims, especially for use in phacoemulsification, **characterised by** a tubular portion (62) at the distal end of the housing (12), which tubular portion (62) is detachable from the remainder of the housing (12) and has at least two ribs (74) formed therein for linear guiding of the acousto-optical waveguide, the tube being divided by the ribs (74) and the waveguide into at least two chambers (64, 66) which extend axially and are isolated with regard to liquid exchange, the first of which chambers (64, 66) is connectable to a suction device and the second of which is connectable to a rinsing device.

17. Surgical instrument according to any one of the preceding claims, **characterised in that** its distal end is constructed in the form of a puncture cannula in the interior of which the acousto-optical waveguide (38) is movably guided in the vibration direction.

## Revendications

1. Instrument chirurgical (10) pour le rayonnement simultané ou intermittent de lumière laser et d'ultrasons, avec un carter (12), un guide d'ondes acousto-optique (38) dans le carter qui sort à l'extérieur à travers une ouverture du carter (12) formée de manière distale et à l'extrémité proximale duquel la lumière laser peut être modulée, un transducteur d'ultrasons (18) à l'intérieur du carter (12) pour engendrer une onde ultrasonore se propageant le long du guide d'ondes (38) et un corps oscillant (28 à 36) relié de manière proximale au transducteur d'ultrasons (18) et de manière distale au guide d'ondes (38) pour la modulation de l'onde ultrasonore dans le guide d'ondes en adaptant l'expansion latérale de l'onde ultrasonore à la section transversale du guide d'ondes (38) et en amplifiant son amplitude, le corps oscillant (28 à 36) ayant un alésage coaxial au guide d'ondes acousto-optique (38) formé au moins à son extrémité distale, sur la paroi duquel est fixé le guide d'ondes acousto-optique (38),
**caractérisé par le fait que** la lumière laser est modulée dans le guide d'ondes acousto-optique (38) par l'intermédiaire d'une connexion fixe du carter et d'un guide d'ondes optique (16) guidé dans le carter (12), que le guide d'ondes optique (16) se développe à travers un alésage s'étendant de manière coaxiale avec le guide d'ondes acousto-optique (38) dans le transducteur d'ultrasons (18) et que les guides d'ondes optique (16) et acousto-optique (38) sont écartés en direction axiale.

2. Instrument chirurgical selon la revendication 1,
**caractérisé par le fait que** le transducteur d'ultrasons (18,56) est un transducteur composite entraîné par voie piézo-électrique avec des masses oscillantes (24 à 36) axiales et que le corps oscillant (28 à 36) forme au moins une partie de la masse oscillante distale du transducteur composite (18,56).

3. Instrument chirurgical selon la revendication 2,
**caractérisé par le fait que** les masses oscillantes (24 à 36) sont fabriquées en titane, un alliage de titane, un alliage d'aluminium ou en silicium cristallin.

4. Instrument chirurgical selon la revendication 2 ou 3,
**caractérisé par le fait qu'**au moins la masse oscillante distale du transducteur composite (18,56) présente plusieurs sections (28 à 36) en direction axiale et qu'au moins une section axiale de la masse oscillante distale du transducteur d'ultrasons (18,56) est réalisée en tant que transformateur d'amplitude (32).

5. Instrument chirurgical selon l'une des revendications précédentes,
**caractérisé par le fait que** le transformateur d'amplitude (32) est un cornet à gradins.

6. Instrument chirurgical selon l'une des revendications précédentes,
**caractérisé par le fait que** le corps oscillant a un élément d'accouplement (36), s'effilant sensiblement en forme de cône en direction de son extrémité distale en direction axiale du guide d'ondes acousto-optique, démontable et fixé par une liaison (44) à fermeture géométrique, dans lequel l'alésage s'étendant depuis la pointe conique le long de l'axe du cône et coaxial au guide d'ondes acousto-optique (38) est conformé pour la réception de sa section terminale proximale.

7. Instrument chirurgical selon l'une des revendications précédentes,
**caractérisé par le fait que** le guide d'ondes acousto-optique (38) est fixé dans la zone de son extrémité proximale à la paroi de l'alésage distal de l'élément d'accouplement (36) à l'aide d'une liaison de collage.

8. Instrument chirurgical selon l'une des revendications précédentes,
**caractérisé par le fait que** la liaison de collage entre le guide d'ondes (38) et les parois de l'alésage est formée par une résine époxy durcissant à chaud avec une température vitreuse élevée et un faible amortissement acoustique.

9. Instrument chirurgical selon la revendication 8,
**caractérisé par le fait que** la température vitreuse de la résine époxy est égale à au moins 100° C.

10. Instrument chirurgical selon l'une des revendications précédentes,
**caractérisé par le fait que** le guide d'ondes acousto-optique (38) est une fibre de verre quartzeux avec un diamètre entre 300 et 1700 micromètres.

11. Instrument chirurgical selon la revendication 10,
**caractérisé par le fait que** le guide d'ondes (38) est entouré par une enveloppe en acrylate, polyacrylate ou polyimide.

12. Instrument chirurgical selon l'une des revendications 1 à 11,
**caractérisé par le fait que** la distance (46) entre les guides d'ondes optique (16) et acousto-optique (38) est comprise entre 50 et 800 micromètres.

13. Instrument chirurgical (48) pour le rayonnement simultané ou intermittent de lumière laser et d'ultrasons, avec un carter (12), un guide d'ondes optique (38) dans le carter qui sort vers l'extérieur à travers une ouverture du carter (12) formée de manière distale et à l'extrémité proximale duquel la lumière laser peut être modulée, un transducteur d'ultrasons (56) à l'intérieur du carter (12) pour engendrer une onde ultrasonore se propageant le long du guide d'ondes (38) et un corps oscillant (28 à 36) relié de manière proximale au transducteur d'ultrasons (56) et de manière distale au guide d'ondes (38) pour la modulation de l'onde ultrasonore dans le guide d'ondes en adaptant l'expansion latérale de l'onde ultrasonore à la section transversale du guide d'ondes (38) et en amplifiant son amplitude, le corps oscillant (28 à 36) ayant un alésage coaxial au guide d'ondes acousto-optique (38) formé au moins à son extrémité distale, sur la paroi duquel est fixé le guide d'ondes acousto-optique (38),
**caractérisé par le fait qu'**un laser (50) est disposé de manière proximale à l'intérieur du carter et engendre un rayon lumineux (52) coaxial au guide d'ondes acousto-optique (38) et qu'un guide d'ondes optique est guidé à travers le transducteur d'ultrasons (56), à l'extrémité proximale duquel la lumière laser est modulée à l'aide d'une lentille convergente (54), les guides d'ondes optique et acousto-optique (38) étant écartés en direction axiale.

14. Instrument chirurgical selon l'une des revendications précédentes,
**caractérisé par le fait que** le transducteur d'ultrasons (18,56) est coulissant, en direction axiale du guide d'ondes acousto-optique (38), à l'intérieur du carter (12).

15. Instrument chirurgical selon la revendication 14,
**caractérisé par** un moteur linéaire (58) pour l'entraînement du déplacement axial du transducteur d'ultrasons.

16. Instrument chirurgical selon l'une des revendications précédentes, en particulier pour l'utilisation dans le cas de l'émulsion pharmacologique,
**caractérisé par** une section (62) de forme tubulaire, séparable du reste du carter (12), à l'extrémité distale du carter (12), avec au moins deux entretoises (74) formées dedans pour le guidage linéaire du guide d'ondes acousto-optique, le tube étant divisé par les entretoises (74) et le guide d'ondes en au moins deux chambres (64,66) s'étendant axialement et isolées vis-à-vis de l'échange de liquides, dont la première peut être reliée à un dispositif d'aspiration et la seconde à un dispositif de rinçage.

17. Instrument chirurgical selon l'une des revendications précédentes,
**caractérisé par le fait que** son extrémité distale est conformée en tant que canule de piqûre dans l'intérieur de laquelle le guide d'ondes acousto-optique (38) est guidé de manière mobile en direction d'oscillation.
